# NEUE EUROPÄISCHE PATENTSCHRIFT

(11) **EP 0 434 793 B2**
(45) Veröffentlichungstag und Bekanntmachung des Hinweises auf die Entscheidung über den Einspruch: **29.01.2003**
(45) Hinweis auf die Patenterteilung: 05.04.1995
(21) Anmeldenummer: 90909441.9
(22) Anmeldetag: 28.06.1990
(51) Int. Cl.: A61B 1/04, A61B 1/00

(54) **ENDOSKOP MIT EINER AM DISTALEN ENDE ANGEORDNETEN VIDEOEINRICHTUNG**
ENDOSCOPE WITH A VIDEO DEVICE ARRANGED AT ITS DISTAL END
ENDOSCOPE AVEC DISPOSITIF VIDEO AGENCE A SON EXTREMITE DISTALE

(30) Priorität: 28.06.1989 DE 3921233
(43) Veröffentlichungstag der Anmeldung: 03.07.1991
(73) Patentinhaber: Karl Storz GmbH & Co. KG, 78532 Tuttlingen (DE)
(72) Erfinder: IRION, Klaus, D-7206 Emmingen-Liptingen (DE)
(74) Vertreter: Harrison, Michael Charles
(86) Internationale Anmeldenummer: DE9000486
(87) Internationale Veröffentlichungsnummer: WO91000049

(56) Entgegenhaltungen:
- DE-A- 3 618 906
- DE-A- 3 806 190
- DE-U- 7 833 379
- JP-A- 60 182 928

## Beschreibung

### Technisches Gebiet

Die Erfindung bezieht sich auf ein Endoskop mit einer am distalen Ende eines Endoskop-Schaftes angeordneten Videoeinrichtung, die mittels eines Übertragungssystems mit einer proximal angeordneten Versorgungseinheit verbunden ist, und die ein Objektiv aufweist, das ein mittels einer Beleuchtungseinheit beleuchtetes Objektfeld auf einen Bildaufnehmer abbildet, wobei das Objektiv und der Bildaufnehmer zu einer Videoeinheit zusammen gefaßt sind.

Endoskope dienen der Beobachtung von Hohlräumen zu Untersuchungs- und/oder Manipulations-Zwecken und haben sowohl in der Technik als auch in der Medizin eine Vielzahl von Anwendungen gefunden.

### Stand der Technik

Herkömmliche Endoskope weisen einen sogenannten Bild-Weiterleiter auf, der das von einem am distalen Ende angeordneten Objektiv erzeugte Bild vom distalen zum proximalen Ende "weiterleitet", an dem es mittels eines Okulars betrachtet wird. Der Bild-Weiterleiter besteht bei "starren" Endoskopen aus sogenannten Relais-Linsensätzen und bei flexiblen Endoskopen aus Faser-Bündeln.

Seit einiger Zeit sind nun Video-Bildaufnehmer, wie beispielsweise CCD-Chips, mit geringen Abmessungen verfügbar. Es ist deshalb mehrfach vorgeschlagen worden, anstelle der Verwendung des Bild-Weiterleiters in der Bildebene des distal angeordneten Objektivs einen Bildaufnehmer anzuordnen, der mittels eines Übertragungssystems mit einer proximal angeordneten Versorgungseinheit verbunden ist. Hierzu wird exemplarisch auf die US-Patentschriften 4 253 447 oder 4 261 344 verwiesen.

Bei diesen Vorschlägen ist in der Bildebene des distal angeordneten Objektivs ein "stehender" Halbleiter-Bildaufnehmer, d.h. ein Bildaufnehmer angeordnet, der mit der Achse des Endoskops einen 90°-Winkel einschließt.

Bei einer derartigen Anordnung des Bildaufnehmer-Chips ergeben sich jedoch insbesondere bei medizinischen Endoskopen Probleme, da gegenwärtig Bildaufnehmer-Chips verglichen mit den Abmessungen medizinischer Endoskope noch relativ groß sind. Insbesondere sind die Abmessungen des Bildaufnehmer-Chips störend bei Endoskopen, die nicht nur der Beobachtung eines Hohlraums dienen, sondern die zusätzlich auch eine Manipulation in dem Hohlraum ermöglichen sollen, und hierzu einen vom distalen zum proximalen Ende führenden Hauptkanal aufweisen, in den beispielsweise Scheren, Pinzetten etc. einsetzbar sein sollen. Um das für den Hauptkanal verfügbare Lumen durch den Bildaufnehmer-Chip nicht zu stark einzuschränken, ist beispielsweise in den deutschen Offenlegungsschriften 35 29 026 und 37 20 624 vorgeschlagen worden, den Bildaufnehmer-Chip "liegend", d.h. parallel zur Längsachse des Endoskops anzuordnen.

Sieht man einmal von dieser speziellen Anordnung des Bildaufnehmer-Chips im distalen Endstück des Endoskops ab, so unterscheiden sich die bisher vorgeschlagenen "Video-Endoskope" von herkömmlichen Endoskopen nur dadurch, daß das Bildleiter-System, also beispielsweise das Relais-Linsensystem durch einen Bildaufnehmer mit nachgeordnetem elektrischen Übertragungssystem ersetzt ist. Der restliche Aufbau des Endoskops, also beispielsweise die Anordnung des distal vorgesehenen Objektivs, der Beleuchtungseinheit sowie die Anordnung der gegebenenfalls vorgesehenen Kanäle für die Pinzetten, Scheren und dgl. sind dagegen praktisch unverändert.

Damit ergeben sich durch die Verwendung eines Bildaufnehmers anstelle eines Bildleiter-Systems keine neuen Beobachtungsmöglichkeiten und damit auch beispielsweise in der Medizin keine neuen Behandlungsmöglichkeiten.

Weiterhin bedeutet die beim Stand der Technik vollzogene einfache Ersetzung eines Bildleiter-Systems durch einen Bildaufnehmer, daß weiterhin das Gesamt-Lumen des Endoskops, d.h. die erforderliche Querschnittsfläche im wesentlichen durch die "Addition" der verschiedenen Lumen gegeben ist, die für das unverändert eingebaute Objektiv, die Kanäle für Spülflüssigkeiten, Pinzetten, Scheren etc. erforderlich sind.

Eine Vorrichtung mit den Merkmalen des Oberbegriffs der unabhängigen Ansprüche ist in der Offenlegungsschrift JP 64-83238 offenbart.

### Darstellung der Erfindung

Der Erfindung liegt die Aufgabe zugrunde, ein Endoskop mit einer am distalen Ende angeordneten Videoeinrichtung derart weiterzubilden, daß sich neue Beobachtungs- und Behandlungsmöglichkeiten ergeben, und daß insbesondere das Gesamt-Lumen des Endoskops nicht mehr durch die Addition der verschiedenen, für die einzelnen Bauteile, wie Objektiv, Spül- und Manipulationskanäle, Beleuchtungseinrichtung etc. erforderlichen Einzellumen gegeben ist.

Die Erfindung geht von dem Grundgedanken aus, daß durch die Verwendung eines distal angeordneten Bildaufnehmers beim Aufbau eines Endoskops wesentlich mehr gestalterische Möglichkeiten als bei herkömmlichen Endoskopen mit "fest integriertem bildgebenden System gegeben sind, gleichgültig, ob diese zur Bildübertragung vom distalen zum proximalen Ende eine Videoeinrichtung oder einen Bild-Weiterleiter aufweisen.

Eine erfindungsgemäße Lösung dieser Aufgabe ist im Anspruch 1 gekennzeichnet. ErfindungsgemäB ist die Videoeinheit derart beweglich mit dem Endoskop-Schaft verbunden, daß die Querschnitts-Außenkontur der Videoeinheit beim Einführen in den zu beobachtenden Hohlraum im wesentlichen innerhalb der Querschnitts-Außenkontur des distalen Endes des Endoskop-Schaftes liegt, und daß nach Beendigung des Einführvorganges die Videoeinheit relativ zum distalen Ende des Endoskop-Schafts derart bewegbar ist, daß Querschnitts- und/oder Längsschnitts-Außenkontur der Videoeinheit über die entsprechende Außenkontur, des Endoskop-Schaftes hinaus bewegt wird, wobei die Videoeinheit um eine zur Achse des Endoskop-Schaftes parallele und bezogen auf die Stirnfläche der Videoeinheit exzentrische Achse schwenkbar ist.

Der Vorteil dieser lösung besteht darin, das distale Video-Beobachtungssystem nicht mehr als fest mit dem Endoskop verbundene und in die Endoskop-Struktur integrierte Teile auszubilden, wie dies beim Stand der Technik der Fall ist, sondern das Objektiv und den Bildaufnehmer sowie gegebenenfalls die Beleuchtungseinheit für das Objektfeld des Objektivs zu einer Videoeinheit zusammenzufassen, die nach dem Einführen in den zu beobachtenden Hohlraum als Ganzes gegenüber dem distalen End des Endoskop-Schafts bewegbar ist. Dabei kann die Videoeinheit aus dem Endoskop-Schaft bzw. bei einer Anordnung "vor dem Schaft" aus der "Querschnitts-Kontur" des Endoskop-Schaftes "ausgeschwenkt" werden. Dies ermöglicht nicht nur eine Beobachtung des Hohlraums unter einem anderen Blickwinkel, sondern hat vor allem bei einem Endoskop mit wenigstens einem Hauptkanal für Spülflüssigkeiten, Instrumente etc. den Vorteil einer wesentlich besseren Ausnutzung des verfügbaren Lumens, so daß die Einzellumen, d.h. die Querschnittsflächen der einzelnen Bauteile zusammengenommen größer sind als die gesamte Querschnittsfläche des Endoskogs während des Einführvorgangs in den Hohlraum.

Dieser Vorteil kann sowohl bei einem sog. starren Endoskop als auch bei einem flexiblen Endoskop realisiert werden.

Die erfindungsgemäße Ausbildung führt zu einem Video-Endoskop, das gegenüber bekannten Endoskopen mit einer am distalen Ende "starr" angeordneten, d.h. fest in die Struktur des Endoskops bzw. des Endoskop-Schaftes integrierte Videoeinrichtung eine Reihe von Vorteilen aufweist:

Beispielsweise, wird (beispielsweise) während des Einführ- und Entnahmevorgangs in bzw. aus dem Hohlraum die Videoeinheit so angeordnet, daß sie die Kanalöffnung wenigstens teilweise verdeckt. Nach dem Einführen in den Hohlraum wird die Videoeinheit in die Beobachtungsstellung überführt, in der sie den Hauptkanal freigibt. Damit ist der Querschnitt des Endoskops nicht mehr durch die Addition der für den oder die Kanäle erforderlichen Querschnittsflächen und die Querschnittsfläche des Objektivs nebst Videoeinheit gegeben, sondern nur noch durch das größte Lumen der verschiedenen Einzel-Lumen.

Bei einer Weiterbildung ist dabei der Querschnitts-Außendurchmesser der Videoeinheit annähernd so groß wie der des Endoskop-Schaftes. Dies ermöglicht eine vergleichsweise große Videoeinheit und damit die Verwendung eines großen und somit lichtstarken Objektivs sowie den Einsatz eines Bildaufnehmers, beispielsweise eines Festkörper-Bildwandlers mit einer großen lichtempfindlichen Fläche, ohne daß der Durchmesser des Endoskops während des Einführ- und Entnahmevorgangs unannehmbar groß würde.

Die Schwenkbewegung der am distalen Ende des Endoskops angelenkten Videoeinheit kann in unterschiedlicher Weise, beispielsweise mit mikromechanischen Aktuatoren, vom distalen zum proximalen Ende verlaufenden Achsen etc. ausgeführt werden. Bei einer bevorzugten Ausgestaltung ist zur Durchführung der Schwenkbewegung ein vom proximalen zum distalen Ende und zurück verlaufender Seilzug vorgesehen. Diese Ausführung hat den Vorteil, daß der Seilzug leicht in "nicht genutzten" Bereichen des Querschnitts der Schaftes geführt werden kann.

Gemäß einem weiteren Ausführungsbeispiel weist der Endoskop-Schaft (wenigstens) einen Übertragungskanal auf, in dem die die Schwenkbewegung der Videoeinheit erzeugenden Elemente, beispielsweise die bereits angepsrochene Achse, die im folgenden auch Bewegungselemente genannt werden, sowie gegebenenfalls das Übertragungssystem für die Videosignale vom distalen zum proximalen Ende geführt sind.

Dabei ist es bevorzugt, wenn der Übertragungskanal durch einen in Richtung der Achse des Endoskop-Schaftes verlaufenden Schlitz mit dem Hauptkanal verbunden ist. Dies erlaubt nicht nur eine Entnahme des Übertragungssystems aus dem Übertragungskanal und damit ein Trennen der Videoeinheit vom eigentlichen Endoskop, sondern bei der Weiterbildung, gemäß der die Querschnitts-Außenkontur der Videoeinheit an die Innenkontur des Hauptkanals angepaßt ist, ein Vorschieben der Videoeinheit vom proximalen Ende zum distalen Ende bzw. umgekehrt ein Zurückschieben der Einheit. Damit kann beispielsweise die Videoeinheit gegen eine andere Videoeinheit, die beispielsweise ein Objektiv mit einer anderen Brennweite und damit ein anderes Gesichtsfeld hat, oder gegen ein anderes Beobachtungs- oder Behandlungssystem, beispielsweise eine herkömmliche Beobachtungsoptik mit einem Objektiv und Bildleitern, ausgetauscht werden, ohne daß der Endoskop-Schaft aus dem Hohlraum entnommen werden müßte.

Da durch die erfindungsgemäße Ausbildung die Videoeinheit zumindest den größten Teil des Hauptkanals freigibt, kann in den Hauptkanal aber auch eine zusätzliche herkömliche Beobachtungsoptik mit einem Bildleitersystem eingesetzt werden.

In jedem Falle ist es bevorzugt, wenn der Übertragungskanal auch als Führung für das bzw. die Elemente dient, die die Bewegung der Videoeinheit erzeugen.

Selbstverständlich ist es aber auch möglich, das Bewegungselement im Hauptkanal anzuordnen. Diese Anordnung ist insbesondere dann von Vorteil, wenn eine als Bewegungselement dienende Schubstange an der Videoeinheit exzentrisch bezogen auf den Querschnitt der Videoeinheit angebracht ist. Diese Ausbildung hat den Vorteil, daß nach dem Vorschieben der Videoeinheit über das distale Ende des Schaftes hinaus, die Videoeinheit ohne weitere Maßnahmen allein durch die Schwerkraft in eine Stellung überführt wird, in der sie den größten Teil des Kanalquerschnitts freigibt.

Eine Weiterbildung, bei der die Längsschnitts-Kontur der Videoeinheit derart ausgebildet ist, daß sich ein kantenfreier und weicher Übergang vom maximalen Querschnitt der Videoeinheit zum Querschnitt der Schubstange ergibt, stellt sicher, daß durch einfaches Zurückziehen der Schubstange die Videoeinheit wieder in den Hauptkanal zurückziehbar ist, so daß ein problemloses Entnehmen des Endoskops möglich ist.

In jedem Falle ist es im Hinblick auf die opimale Ausnutzung des verfügbaren Lumens von Vorteil, wenn das Bewegungselement, also beispielsweise die Achse oder die Schubstange, hohl ist und im Bewegungselement das Übertragungssystem geführt ist. Das Bewegungselement kann dabei je nach Ausbildung des Endoskops (starr oder flexibel) beispielsweise ein starres Hohlrohr oder eine biegsame Welle sein. Weiterhin können auch in einem herkömmlichen flexiblen Endoskop benötigte "Züge" mit einer weiteren Funktion versehen werden, so daß beispielsweise durch zusätzliches "Drehen" dieser Züge der Schwenkvorgang erfolgt. Ferner kann auch ein durchsichtiger Kunststoffzylinder, der gleichzeitig als Lichtleiter für Beleuchtungslicht dient, als Element zur Übertragung der Drehbewegung eingesetzt werden.

Durch eine Ausbildung, gemäß der die Videoeinheit in der Ruhestellung, d.h. in der Stellung, in der das Endoskop in den Hohlraum eingeführt wird bzw. aus diesem entnommen wird, vollständig in den Schaft einschwenkbar ist, wird die Videoeinheit optimal vor Beschädigungen geschützt.

Daß wenigstens das Objektiv und den Bildaufnehmer zu einer kompakten Einheit zusammengefaßt sind, die nach dem Einführen in den Hohlraum "aus dem Endoskop-Schaft" ausschwenkbar ist, ermöglicht es darüberhinaus, nicht nur eine schwenkbare Einheit, sondern mehrere schwenkbare Einheiten vorzusehen, von denen wenigstens eine eine Videoeinheit ist.

Dabei ist es bevorzugt, wenn die bewegbaren und insbesondere schwenkbaren Einheiten zumindest in der "eingeschwenkten Stellung" hintereinander am Endoskopschaft angeordnet sind, da dann das für den Einführvorgang und den Entnahmevorgang verfügbare Lumen optimal ausgenutzt wird. Weiterhin ist es von Vorteil, wenn die Einheiten nach dem Ausschwenken in einer auf der Endoskop-Längsachse senkrecht stehenden Ebene angeordnet sind. Dies kann beispielsweise dadurch realisiert werden, daß zunächst sämtliche Einheiten hintereinander angeordnet und "in den Endoskop-Schaft eingeschwenkt" sind. Nach dem Ausschwenken der Einheiten werden die hinter der vordersten Einheit angeordneten Einheiten durch Verschieben ihrer Bewegungselemente in den ihnen jeweils zugeordneten Übertragungskanälen nach vorne so weit verschoben, bis sie sich in der gleichen Ebene wie die vorderste Einheit befinden. Entsprechend ist es nalürlich auch möglich, die vorderen Einheiten entsprechend zurückzuziehen.

Diese Ausbildung dabei eine Reihe von Vorteilen:
Verwendet man für sämtliche Einheiten Videoeinheiten, so wird eine Stereo-Betrachtung mit einer vergleichsweise großen Stereo-Basis möglich. Entsprechend wäre es natürlich auch möglich, mit mehr als zwei Videoeinheiten eine weitergehende redundante Tiefenanalyse des zu beobachtenden Hohlraums durchzuführen.

Weiterhin ist es möglich, daß lediglich eine Einheit eine Videoeinheit ist, und die andere Einheit einen Lichtsender aufweist. Damit kann beispielsweise ein Triangulationsmeßverfahren realisiert werden.

Ferner ist es möglich, daß zusätzlich zur Videoeinheit ein anderer bildgebender Aufnehmer, beispielsweise ein Ultraschall-Bildaufnehmer, verwendet wird.

Eine weitere erfindungsgemäße Lösung der Aufgabe ist im Anspruch 21 gekennzeichnet.

Eine kompakte, baulich nicht in den Endoskop-Schaft integrierte Videoeinheit zu schaffen, erlaubt darüberhinaus auch ein Lösen der Videoeinheit vom distalen Ende des Endoskop-Schaftes. Dabei bleibt die Videoeinheit lediglich über das Übertragungssystem mit der proximalen Versorgungseinheit verbunden, so daß sie als Video-Sonde im Hohlraum verwendbar ist.

Dabei ist es von Vorteil, wenn die als Video-Sonde ausgebildete Videoeinheit vor dem Beginn des Entnahme-Vorgangs wieder am Endoskop-Schaft anbringbar ist. Weiter ist es bevorzugt, wenn die Video-Sonde nach vorne aus dem Endoskop-Schaft herausschiebbar ist . Bei entsprechender Ausführung des "proximalen Endteils" der Videosonde kann diese aber auch ohne Instrument durch einfaches "Herausziehen" wieder aus dem Hohlorgan entfernt werden.

Eine nach weitere erfindungsgemäße Lösung der Aufgabe ist im Anspruch 24 gekennzeichnet. Damit ist es möglich, beispielsweise eine "zunächst liegend am Schaft" angeordnete Videoeinheit nach dem Einführen in den Hohlraum "aufzustellen".

Ferner ist es möglich, ein zu beobachtendes Objekt unter verschiedenen Blickwinkeln aufzunehmen.

Die verschiedenen in den Ansprüchen gekennzeichneten Bewegungsmöglichkeiten können einzeln oder in Kombination verwendet werden: So ist es möglich, die Videoeinheit beispielsweise zunächst um eine zur Achse des Endoskop-Schaftes parallele und bezogen auf die Stirnfläche der Videoeinheit exzentrische Achse aus dem Endoskop-Schaft auszuschwenken und anschließend durch ein teleskopartig ausgebildetes Bewegungselement in Richtung des Endoskop-Schaftes zu verschieben. Darüberhinaus kann auch eine Verschiebung quer oder schräg zur Achse sowie eine Drehung der Videoeinheit um eine zur Endoskop-Achse senkrechte Achse vorgesehen werden. Selbstverständlich ist es nicht nur möglich, die Bewegungen mit mechanischen Übertragungselementen zu realisieren. Die Bewegungen können auch durch entsprechend ausgebildete Antriebselemente, beispielsweise mikromechanisch hergestellte Motoren etc., realisiert werden.

Die als vom eigentlichen Endoskop getrennte Einheit aufgebaute Videoeinrichtung kann dabei in an sich bekannter Weise aufgebaut sein: Insbesondere ist es möglich, daß der Bildaufnehmer senkrecht zur Achse des Endoskop-Schaftes und parallel zur Achse des Endoskop-Schaftes angeordnet ist. Ferner ist es auch möglich, in einer Videoeinheit einen Bildaufnehmer mit zwei Objektiven vorzusehen, deren Bild wahlweise auf den Bildaufnehmer gerichtet werden kann. Diese Ausbildung ermöglicht beispielsweise bei einer 180°-Anordnung der Objektive die Beobachtung eines wesentlich größeren Objektfeldes als es bei herkömmlichen Objektiven möglich ist. Auch können als Objektive sogenannte Gerade-Blick-Objektive als auch sog. Schräg-Blick-Objektive verwendet werden.

Da für die Videoeinheit beim Einführ-und Entnahmevorgang ein wesentlich größeres Lumen zur Verfügung steht als bei herkömmlichen Video-Endoskopen, ist es darüherhinaus auch möglich, für jeden Farbauszug, d.h. für jede Grundfarbe einen Bildwandler vorzusehen, wobei ein dichroitisches Umlenksystem das Bild des Objektivs entsprechend aufteilt.

Ferner ist es selbstverständlich möglich, die Ausgangssignale des Bildaufnehmer-Chips "drahtlos" an das proximale Ende zu übertragen. Bevorzugt ist es jedoch im Hinblick auf die Baugröße, wenn das Übertragungssystem durch Verbindungskabel für die elektrische und/oder optische Übertragung von Energie und Signalen realisiert wird. Dabei können die einzelnen Leitungen bevorzugt zu einem einzigen Verbindungskabel zusammengefaßt sein. Das Verbindungskabel kann dabei zum Vorschieben der Sonde über den Endoskop-Schaft und zum Zurückziehen der Sonde benutzt werden.

Die Lichtaustrittsfläche der Beleuchtungseinheit kann dabei entweder "fest" am Endoskop-Schaft oder an der Videoeinheit angeordnet sein.

Es ist möglich, in der Videoeinheit eine oder mehrere Miniatur-Glühlampen, Stroboskoplampen, Leuchtdioden und/oder Halbleiterlaser vorzusehen. Natürlich ist es aber auch möglich, in herkömmlicher Weise die Beleuchtungslichtquelle proximal anzuordnen, und das Licht der Beleuchtungslichtquelle mittels Uchtleiter zur Lichtaustrittsfläche zu leiten, wo es dann austritt. Die Lichtleiter können dabei herkömmliche Faserbündel, aber auch starre Stäbe sein, die gleichzeitig als Bewegungselemente dienen.

Weiterhin ist es möglich, die Energieversorgung des Bildaufnehmers sowie gegebenenfalls der Beleuchtungslichtquelle dadurch zu realisieren, daß in der Videoeinheit optoelektrische und/oder elektromagnetische Wandler vorgesehen sind, die optische oder hochfrequente elektrische bzw. induktiv eingekoppelte Energie in für den Bildaufnehmer geeignete elektrische Energie wandeln. Umgekehrt kann auch das Ausgangssignal der Videoeinheit durch einen elektrooptischen Signalwandler in ein optisches Signal umgesetzt und dieses zum proximalen Ende übertragen werden.

Eine Weiterbildung, gemäß der der dem proximalen Ende zugekehrte Endbereich der Videoeinheit sich stetig verjüngt, und der distale Endbereich des Endoskop-Schaftes komplementär hierzu ausgebildet ist, ermöglicht bei der Version, bei der die Videoeinheit über den distalen Endbereich vorschiebbar ist, ein sicheres "selbstzentrierendes" Einsetzen der Videoeinheit bzw. Videosonde in den Endoskop-Schaft vor Beginn des Entnahme-Vorgangs bzw. eine Entnahme der Videosonde ohne vorheriges Anbringen an dem Endoskop-Schaft.

Durch eine Weiterbildung eines flexiblen Endoskop-Schaftes, gemäß der fluidbeaufschlagbare Kammern vorgesehen sind, die bei einer Beaufschlagung den Endoskop-Schaft gegen Querkräfte versteifen, ist es möglich, den flexiblen Endoskop-Schaft so zu versteifen, daß eine Verschiebung des Elements quer zum Endoskop-Schaft möglich wird. Darüberhinaus kann durch eine Teilbeaufschlagung der Kammern eine Abwinklung des Endoskpp-Schaftes erfolgen.

Die im Anspruch 40 gekennzeichnete Ausbildung ermöglicht eine Trennung der Videoeinheit vom Endoskop-Schaft.

Das im Anspruch 41 vorgesehene magnetisch beeinflußbare Element ermöglicht eine Positionierung der Videosonde unabhängig vom Endoskop-Schaft, also beispielsweise eine Positionierung von außerhalb des Körpers.

Die im Anspruch 42 vorgesehenen Verbindungskabel weisen Stecker auf, deren Querschnittsabmessungen kleiner oder gleich den Abmessungen der Verbindungskabel sind, die wiederum wesentlich kleiner als die maximalen Querschnittsabmessungen der Videoeinheit sind, so daß eine Trennung der Videoeinheit vom Schaft durch einfaches Herausziehen des Verbindungskabels möglich ist.

Gegebenenfalls kann die Videoeinheit auch einen Spülkanal aufweisen, der beispielsweise zum Säubern der Objektiv-Frontlinse verwendet werden kann.

Der Querschnitt des Endoskop-Schaftes kann selbstverständlich wie bei bekannten Schäften rund sein, wobei der Querschnitt der Videoeinheit rund sein kann, aber nicht sein muß. Es ist jedoch auch möglich, den Querschnitt des Schaftes und der Video-Einheit gleich, aber nicht rund, sondern beispielsweise oval auszubilden. Die Freigabe von im Schaft vorgesehenen Kanälen, wie beispielsweise Pinzetten-Kanälen kann dann durch eine Drehung der Videoeinheit um 90° relativ zum Schaft erfolgen.

### Kurze Beschreibung der Zeichnung

Die Erfindung wird nachstehend anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird.

Es zeigen:
Fig. 1a und 1b
   einen Längsschnitt durch ein erstes Ausführungsbeispiel,
Fig. 1c
   eine Aufsicht auf dieses Ausführungsbeispiel,
Fig. 1d und 1e
   die die Schwenkbewegung ausführenden Bewegungselemente,
Fig. 2
   einen Längsschnitt durch ein zweites Ausführungsbeispiel,
Fig. 3
   einen Längsschnitt durch ein drittes Ausführungsbeispiel,
Fig. 4
   einen Längsschnitt durch ein viertes Ausführungsbeispiel,
Fig. 5a und 5b
   zwei Varianten eines fünften Ausführungsbeispiels in Aufsicht,
Fig. 6a bis 6c
   ein sechstes Ausführungsbeispiel in verschiedenen Stellungen,
Fig. 7
   einen Längsschnitt durch ein siebtes Ausführungsbeispiel,
Fig. 8a und 8b
   einen Längsschnitt durch ein neuntes Ausführungsbeispiel in zwei Stellungen.

### Darstellung von Ausführungsbeispielen

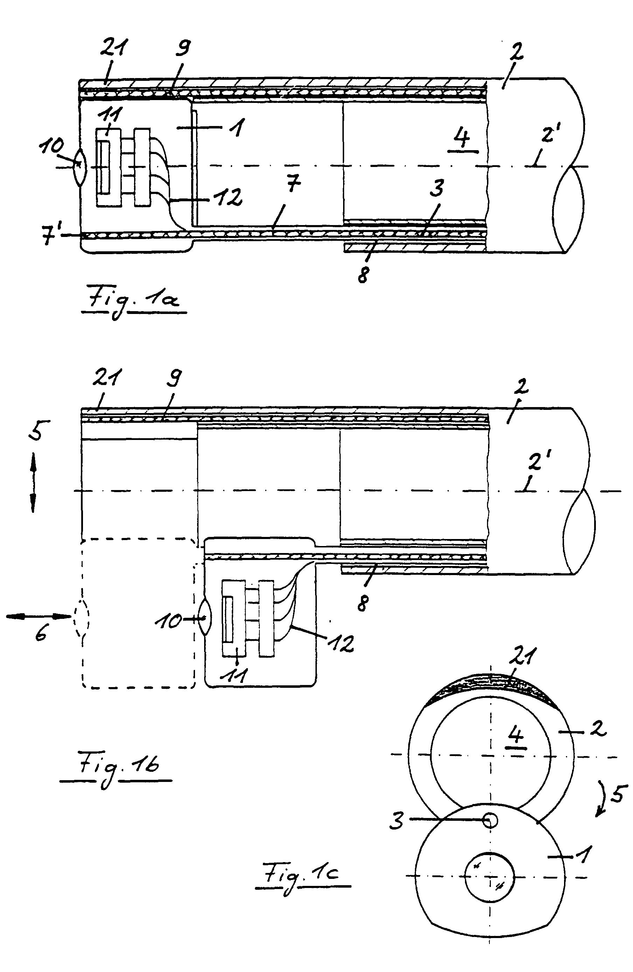

Fig. 1 zeigt ein erstes Ausführungsbeispiel der Erfindung, bei dem eine Videoeinheit 1 um eine zur Achse 2' des Endoskop-Schaftes 2 parallele Achse 3 schwenkbar ist. Der Endoskop-Schaft 2, der bei dem dargestellten Ausführungsbeispiel lediglich exemplarisch als starrer Schaft dargestellt ist, weist in an sich bekannter Weise einen Hauptkanal 4 auf, der beispielsweise zum Spülen mit Spülflüssigkeiten verwendet werden kann, oder in den Instrumente wie Scheren, Pinzetten etc. einsetzbar sind. Der Hauptkanal 4 verbindet das in Fig. 1 dargestellte distale Ende, d.h. das in den zu beobachtenden Hohlraum einsetzbare Ende des Schaftes 2 mit dem in Fig. 1 nicht dargestellten proximalen Ende, d.h. dem außerhalb des Hohlraums verbleibenden Ende.

Da die Schwenkachse 3 der Videoeinheit 1 bezogen auf die Stirnfläche der Videoeinheit exzentrisch angeordnet ist, kann die Videoeinheit 1 aus der in Fig. 1 a dargestellten Stellung, in der sie in den Hauptkanal 4 des Endoskop-Schaftes eingeschwenkt ist, in Richtung eines Pfeils 5 in eine Stellung geschwenkt werden, in der sie den Hauptkanal 4 vollständig freigibt (Fig. 1b). Die Abmessungen der Videoeinheit 1 und des Schaftes 2 sowie die Anordnung der Schwenkachse 3 sind dabei so gewählt, daß die Außenkontur der Videoeinheit im eingeschwenkten Zustand bei einer Betrachtung in Richtung der Längsachse vollständig innerhalb der Außenkontur des Schaftes liegt. Erst nach einer Drehung der Videoeinheit um die Schwenkachse 3 ragt die "Querschnitts-Außenkontur" der Videoeinheit über die entsprechende Kontur des Schaftes hinaus.

Zusätzlich ist die Videoeinheit 1 längs eines Pfeiles 6, d.h. parallel zur Achse 2' verschiebbar. Die ermöglicht nicht nur eine Veränderung des Objektfeldes der Videoeinheit, sondern auch den Einsatz von Instrumenten, wie Pinzetten, Scheren in den Hauptkanal 4, die am distalen Ende sehr stark abgewinkelt werden können, ohne daß der Abwinkelvorgang durch die Videoeinheit behindert werden würde. Selbstverständlich ist es aber auch möglich, in den Hauptkanal 4 eine herkömliche Endoskop-Optik mit einem Objektiv und einem Bild-Übertragungssystem als zusätzliches Beobachtungssystem einzusetzen.

Zur Realisierung der Schwenk- und Verschiebebewegung ist an der Videoeinheit ein Element 7 befestigt, das in einem Übertragungskanal 8, der am Außenumfang des Schaftes 2 vorgesehen ist, geführt ist. Bei der in den Fig. 1a bis 1c gezeigten Ausführungsform ist das die Bewegung übertragende Element 7 (Bewegungselement) ein aus einem durchsichtigen Material bestehender Lichtleitstab, der an einer Lichtaustrittsfläche 7' an der Stirnseite der Videoeinheit 1 endet. Zusätzlich sind bei dem dargestellten Ausführungsbeispiel noch weitere Lichtleiter 9 vorgesehen, die "starr" in die Schaftstruktur integriert sind, und die beispielsweise herkömmliche Faserbündel sein können.

Die Fig. 1d und 1e zeigen eine andere Ausführungsform für das die Schwenkbewegung übertragende Bewegungselement 7. Die Videoeinheit 1 ist mittels einer Welle 71 am Schaft 2 um die Achse 3 schwenkbar angelenkt. Zur Drehung der Welle 71 ist ein Seilzug 72 vorgesehen, der vom proximalen zum distalen Ende des Schafts 2 und zurück verläuft, wobei zwei auf beiden Seiten der Welle 71 angeordnete Rollen 73 den Seilzug umlenken. Damit führt eine Verschiebung des Seilzugs 72 durch ein nicht dargestelltes, am proximalen Ende angeordnetes Betätigungselement zu einer Drehung der Videoeinheit um die Achse 3.

Die Videoeinheit 1 weist ein nur schematisch dargestelltes Objektiv 10 und einen ebenfalls nur schematisch dargestellten Bildaufnehmer-Chip 11 auf, der über Verbindungsleitungen 12 mit einer proximal angeordneten Versorgungseinheit verbunden ist. Bei der in den Fig. 1a bis 1c gezeigten Ausführungsform sind die Verbindungsleitungen 12 um den Stab 7 gewickelt. Selbstverständlich ist es aber auch möglich, das Bewegungselement 7 als hohles Rohr auszubilden, in dem die Leitungen 12 sowie gegebenenfalls Lichtleitfasern geführt sind.

Bei der in den Fig. 1a bis 1c dargestellten Auführungsbeispiel kann die Videoeinheit 1 mit dem in ihr vorgesehenen Objektiv 10 und dem Bildaufnehmer 11 sowie der Lichtaustrittsfläche 7' einer Beleuchtungseinheit aus einer Stellung, in der die Videoeinheit im wesentlichen seitlich des Endoskop-Schaftes 2 angeordnet ist, in den Endoskop-Schaft 2 eingeschwenkt werden, der hierzu einen entsprechenden, in Fig. 1c dunkel dargestellten Vorsprung 21, aufweist.

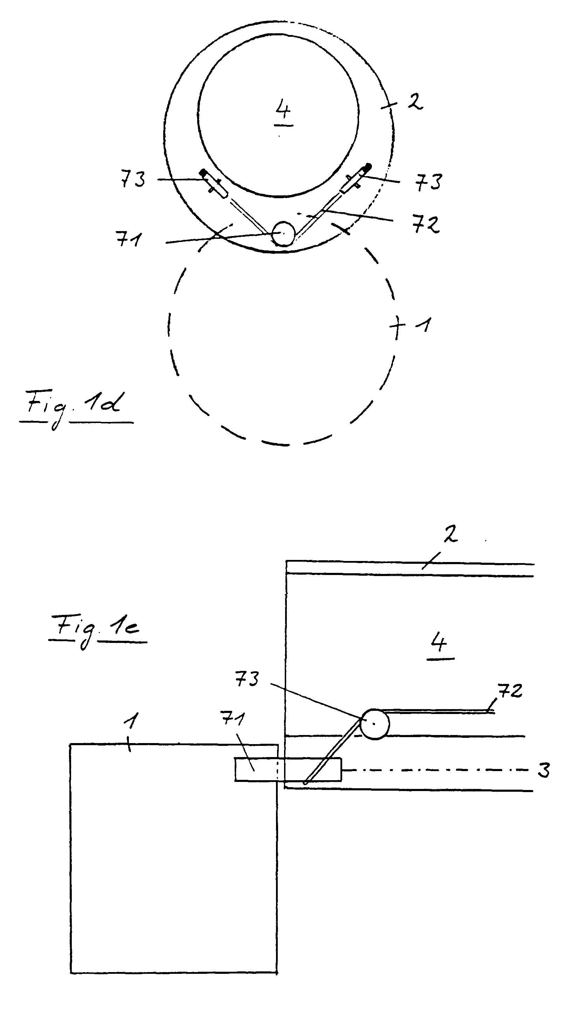

Selbstverständlich ist es aber auch möglich, die Videoeinheit vor dem Endoskop-Schaft 2 anzuordnen und dementsprechend vor den Endoskop-Schaft 2 zu schwenken. Dies zeigt in einer Fig. 1a entsprechenden Darstellung Fig. 2, in der im übrigen gleiche Elemente mit den gleichen Bezugszeichen wie in Fig. 1 versehen sind, so daß auf eine erneute Vorstellung verzichtet werden kann.

Das in Fig. 2 dargestellte Ausführungsbeispiel unterscheidet sich weiter von dem in Fig. 1 dargestellten Ausführungsbeispiel dadurch, daß der Bildaufnehmer 2 nicht senkrecht zur Längsachse 2' des Endoskops, sondern parallel zu dieser angeordnet ist. Entsprechend ist ein Umlenkprisma 13 vorgesehen, das das Bild des Objektivs 10, das bei beiden Ausführungsbeispielen ohne Beschränkung des allgemeinen Erfindungsgedankens ein sog. "Gerade-Blick-Objektiv" ist, auf die lichtempfindliche Fläche des Bildaufnehmers 11 umlenkt. Selbstverständlich ist es aber auch möglich, sog. Schrägblick-Objektive zu verwenden.

Da durch den erfindungsgemäßen Grundgedanken, die Videoeinheit 1 beim Einführ- und Entnahmevorgang des Endoskops vor bzw. in den Hauptkanal 4 zu schwenken, ein wesentlich größeres Lumen für die Videoeinheit als bei herkömmlichen Video-Endoskopen zur Verfügung steht, ist es nicht nur möglich, größere Objektive und größere Bildwandler als bei herkömmlichen Video-Endoskopen zu verwenden, sondern auch einen Aufbau vorzusehen, bei dem drei Bildaufnehmer für die drei Grundfarben vorgesehen sind.

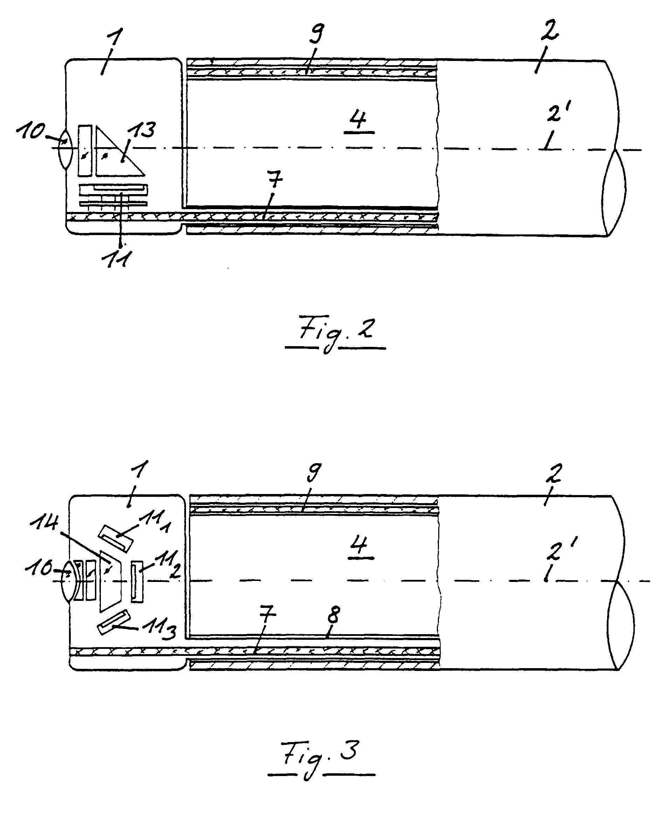

Fig. 3 zeigt eine entsprechende Ausführungsform mit drei Bildwandlern 11₁ bis 11₃. Das Licht des Objektivs 10 teilt ein dichroitisches Bildelement 14 auf die drei Bildwandler 11₁ bis 11₃ auf. Ansonsten entspricht das in Fig. 3 gezeigte dritte Ausführungsbeispiel dem in Fig. 2 dargestellten Ausführungsbeispiel.

Bei den in Verbindung mit den Fig. 1 bis 3 beschriebenen Auführungsbeispielen ist lediglich eine Videoeinheit 1 vorgesehen, die das Objektiv 10, den Bildaufnehmer 11 sowie gegebenenfalls die Lichtaustrittsfläche 7' einer Beleuchtungseinheit aufnimmt, und die nach dem Einführen in den zu beobachtenden Hohlraum als Ganzes gegenüber dem distalen Ende des Endoskop-Schafts 2 bewerbar ist. Selbstverständlich ist es aber auch möglich, mehr als eine Videoeinheit vorzusehen:

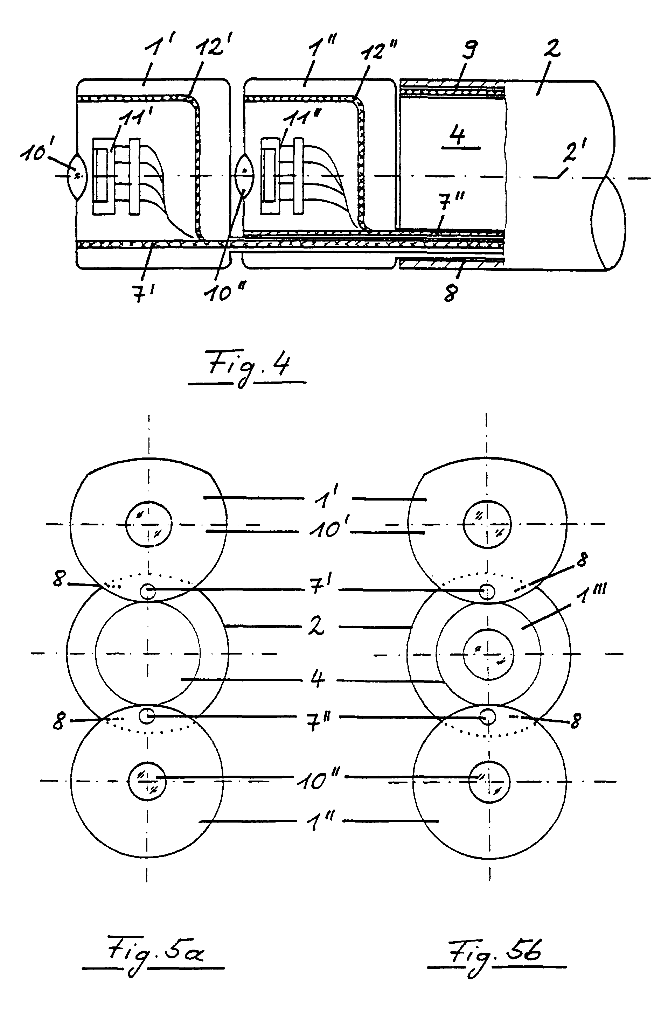

Fig. 4 zeigt ein viertes Ausführungsbeispiel, bei dem zwei Videoeinheiten 1' und 1" vorgesehen sind, deren Bewegungselemente 7' und 7" koaxial in einem Übertragungskanal 8 geführt sind. Ansonsten entsprechen bei dem in Fig. 4 dargestellten Ausführungsbeispiel Elemente mit gleichen Bezugszeichen wie in den vorigen Figuren den dort beschriebenen Elementen, so daß auf eine erneute Vorstellung verzichtet wird.

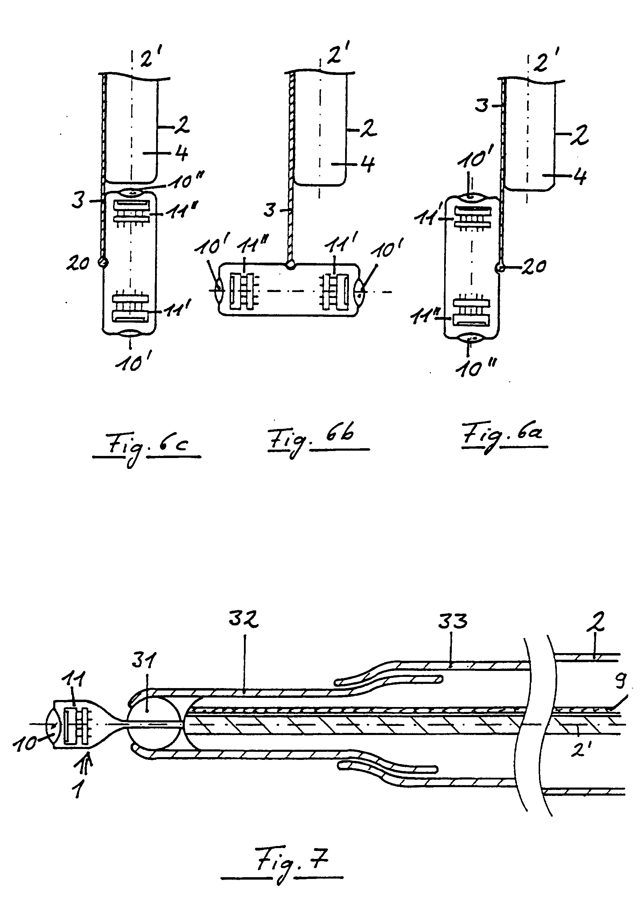

Selbstverständlich ist es aber auch möglich, die Bewegungselemente 7 für die einzelnen Videoeinheiten nicht koaxial, sondern - wie Fig. 5 in einer Aufsicht zeigt - in unterschiedlichen Kanälen 8' und 8" zu führen. Bildet man weiterhin wenigstens eine der beiden Videoeinheiten 1 bzw. 1' in Richtung der Längsachse des Endoskop-Schafts verschiebbar aus, so können die beiden Videoeinheiten 1' und 1" nach dem Ausschwenken in der gleichen Ebene senkrecht zur Endoskop-Längsachse angeordnet werden. Damit ist beispielsweise eine stereoskopische Aufnahme möglich.

Selbstverständlich ist es bei den in den Fig. 4 und 5 dargestellten Ausführungsbeispielen nicht erforderlich, daß beide Einheiten Videoeinheiten sind. Beispielsweise kann eine der beiden Einheiten 1' bzw. 1" eine Lichtquelle, wie beispielsweise eine Miniatur-Glühbirne, eine Leuchtdiode oder eine Laserdiode aufnehmen. Ferner kann auch eine der Einheiten einen anderen bildgebenden Aufnehmer, wie beispielsweise ein Ultraschall-Aray aufnehmen. Letztlich ist es auch möglich, in die Einheiten 1' und 1" ein anderes endoskopisches Meßsystem, wie beispielsweise zwei Laserdioden, zu integrieren und die Beobachtung mit einer in den zentralen Hauptkanal 4 eingeführten Beobachtungseinheit, die beispielsweise ein herkömmliches optisches System 1''' oder eine Videoeinheit sein kann, vorzunehmen (Fig. 5b).

Bei den in Fig. 1 bis 5 dargestellten Ausführungsbeispielen sind die Videoeinheiten 1 um eine zur Achse 2' des Endoskop-Schaftes 2 parallele und bezogen auf die Stirnfläche der Videoeinheit 1 exzentrische Achse 3 schwenkbar. Zusätzlich kann gegebenenfalls eine Verschiebung in Richtung der Achse 3 erfolgen.

Die Fig. 6a bis 6c zeigen ein sechstes Ausführungsbeispiel der Erfindung, bei dem die Videoeinheit 1 um eine zur Achse 2' des Endoskop-Schaftes 2 senkrechte Achse 20 schwenkbar ist. Weiterhin weist bei diesem Ausführungsbeispiel die Videoeinheit 1 zwei Objektive 10' und 10", deren optische Achsen einen 180°-Winkel einschließen, sowie zwei Bildaufnehmer 11' und 11" auf.

In der in Fig. 6a gezeigten "0°-Stellung" ist die Videoeinheit 1 vor den Endoskop-Schaft 2 geklappt, während sie in der in Fig. 6c gezeigten 180°-Stellung parallel zum Endoskop-Schaft 2 liegt und beispielsweise die Öffnung eines nicht näher dargestellten Kanals 4 im Endoskop-Schaft freigibt. Fig. 6b zeigt entsprechend die 90°-Stellung, in der eine Beobachtung der Seitenbereiche beispielsweise eines Operationsgebietes möglich ist.

Zusätzlich kann die Videoeinheit auch um eine zur Achse 2' parallele Achse 3 entsprechend den in den fig. 1 bis 5 dargestellten Ausführungsbeispielen geschwenkt werden.

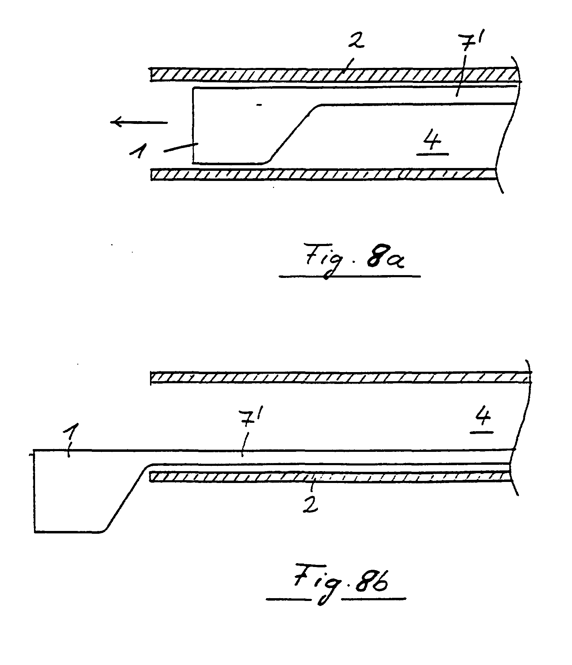

Fig. 7 zeigt ein siebtes Auführungsbeispiel, bei dem die Videoeinheit 1 über ein Kugelgelenk 31 an einem von zwei Elementen 32 und 33 gebildeten Teleskopträger befestigt ist, der wiederum am distalen Ende des Endoskop-Schaftes 2 angelenkt ist. Durch diese Ausführungsform kann die Videoeinheit 1 über das distale Ende des Endoskop-Schaftes 2 hinaus nach vorne vorgeschoben werden und erlaubt damit eine weitergehende Beobachtung. Durch das am vorderen Ende des Teleskops vorgesehene Kugelgelenk 31 ist darüberhinaus eine Beobachtung unter unterschiedlichen Blickwinkeln möglich.

Sämtlichen vorstehend beschriebenen Ausführungsbeispielen ist gemeinsam, daß die Videoeinheit 1 während der Bewegung über entsprechende Elemente mit dem Endoskop-Schaft 2 verbunden bleibt.

Selbstverständlich ist es aber auch möglich, die Videoeinheit 1 derart auszugestalten, daß sie in einer bestimmten Position vom Endoskop-Schaft 2 getrennt werden kann, oder daß sie frei im Kanal 4 eingesetzt ist, wobei die Verbindung zum proximalen Ende durch ein Schubelement erhalten bleibt.

Die Fig. 8a und 8b zeigen ein Ausführungsbeispiel, bei dem die Videoeinheit 1 lediglich in den Hauptkanal 4 des Schafts 2 derart eingesetzt ist, daß sie in diesem verschoben werden kann. An der Videoeinheit 1 ist exzentrisch bezogen auf den Querschnitt der Videoeinheit eine Schubstange 7' angebracht, die ebenfalls im Hauptkanal 4 angeordnet ist. Diese Ausbildung hat den Vorteil, daß nach dem Vorschieben der Videoeinheit über das distale Ende des Schaftes hinaus, die Videoeinheit ohne weitere Maßnahmen allein durch die Schwerkraft in eine Stellung überführt wird, in der sie den größten Teil des Kanalquerschnitts freigibt (Fig. 8b). Bei dem gezeigten Ausführungsbeispiel ist die Längsschnitts-Kontur der Videoeinheit 1 derart ausgebildet, daß sich ein kantenfreier und weicher Übergang vom maximalen Querschnitt der Videoeinheit 1 zum Querschnitt der Schubstange 7' ergibt. Hierdurch ist sichergestellt, daß durch einfaches Zurückziehen der Schubstange die Videoeinheit wieder in den Hauptkanal zurückziehbar ist, so daß in der in Fig. 8a gezeigten Stellung, bei die Videoeinheit 1 im Hauptkanal 4 angeordnet ist, ein problemloses Entnehmen des Endoskops möglich ist.

Vorstehend ist die Erfindung anhand von Ausführungsbeispielen ohne Beschränkung des allgemeinen Erfindungsgedankens beschrieben worden, innerhalb dessen die verschiedensten Modifikationen möglich sind.

Insbesondere können die verschiedenen in den Ausführungsbeispielen dargestellten Bewegungsmöglichkeiten miteinander kombiniert werden:
So ist es möglich, in dem Übertragungskanal 8 ein teleskopartiges Verschiebeelement vorzusehen, an dessen Ende die Videoeinheit um eine zur Achse des Endoskop-Schaftes 2 parallele Achse schwenkbar und um eine hierzu senkrechte Achse drehbar angelenkt ist. Weiterhin ist es möglich, eine Verschiebemöglichkeit der Videoeinheit in einer oder mehreren Richtungen vorzusehen, die mit der Achse des Endoskop-Schaftes einen Winkel ungleich 0° einschließen, also eine "schräge Verschiebung" vorzusehen. Ferner ist es möglich, den Endoskop-Schaft aufklappbar zu gestalten oder mit einem Schlitz zu versehen, so daß die Videoeinheit mitsamt dem an ihr angebrachten Verbindungskabel aus dem Schaft entnommen werden kann. Hierzu ist es auch möglich, einen Schlitz zwischen dem Hauptkanal 4 und dem Übertragungskanal 8 vorzusehen. Die vorstehend beschriebenen Möglichkeiten gelten selbstverständlich gleichermaßen für starre und flexible Endoskope. Bei flexiblen Endoskopen kann es zur Realisierung der verschiedenen Bewegungsmöglichkeiten erforderlich sein, diese durch fluidbeaufschlagbare Kammern "zu ver-steifen", so daß sie semiflexibel werden.

Weiterhin kann es von Vorteil sein, in der am proximalen Ende angeordneten Video-Darstelleinheit durch bekannte Methoden der Bildverarbeitung die distal erfolgte Drehung zu kompensieren, damit sich beispielsweise bei einer Schwenkung der Einheit für den Betrachter nicht "oben" und "unten" vertauschen. Gegebenenfalls kann auch eine mechanische Kompensation durch Drehen des Bildaufnehmers erfolgen.

## Patentansprüche

1. Endoskop mit einer am distalen Ende eines EndoskopSchaftes angeordneten Videoeinrichtung, die mittels eines Übertragungssystems mit einer proximal angeordneten Versorgungseinheit verbunden ist, und die ein Objektiv aufweist, das ein mittels einer Beleuchtungseinheit beleuchtetes Objektfeld auf einen Bildaufnehmer abbildet, wobei das Objektiv (10) und der Bildaufnehmer (11) zu einer Videoeinheit (1) zusammengefaßt sind, wobei die Videoeinheit derart beweglich an dem Endoskop-Schaft gehalten ist, daß die Querschnitts-Außenkontur der Videoeinheit beim Einführen in den zu beobachtenden Hohlraum in wesentlichen innerhalb der Querschnitts-Außenkontur des distalen Endes des Endoskop-Schaftes liegt und nach Beendigung des Einführvorganges die Videoeinheit relativ zum distalen Ende des Endoskop-Schafts über die Querschnitts-Außenkontur hinaus bewegbar ist, **dadurch gekennzeichnet, daß** die Videoeinheit um eine zur Achse (2') des Endoskop-Schaftes parallele und bezogen auf die Stirnfläche der Videoeinheit (1) exzentrische Achse (3) schwenkbar ist.

2. Endoskop nach Anspruch 1,
**dadurch gekennzeichnet, daß** der Querschnitts-Außendurchmesser der Videoeinheit (1) annähernd so groß ist wie der des Endoskop-Schaftes (2).

3. Endoskop nach Anspruch 1 oder 2,
**dadurch gekennzeichnet, daß** die Videoeinheit (1) am distalen Ende des Endoskops rotatorisch auslenkbar angelonkt ist, und daß zur Durchführung der Schwenkbewegung ein vom proximalen zum distalen Ende und zurück verlaufender Seilzug vorgesehen ist, dessen Kraft über ein distales Rollensystem von axial nach lateral umgelenkt wird.

4. Endoskop nach einem der Ansprüche 1 bis 3,
**dadurch gekennzeichnet, daß** der Endoskop-Schaft (2) in an sich bekannter Weise wenigstens einen Hauptkanal (4) für Spülflüssigkeiten, Instrumente etc. aufweist, der das distale mit dem proximalen Ende verbindet, und daß die Videoeinheit (1) während des Einführ- und Entnahmevorgangs des Schaftes in bzw. aus dem Hohlraum die Kanalöffnung wenigstens teilweise verdeckt und in der Beobachtungsstellung den Hauptkanal (4) freigibt.

5. Endoskop nach einem der Ansprüche 1 bis 4,
**dadurch gekennzeichnet, daß** der Endoskop-Schaft (2) einen Übertragungskanal (8) aufweist, in dem die die Schwenkbewegung der Videoeinheit erzeugenden Elemente (Bewegungselemente 7) sowie gegebenenfalls das Übertragungssystem (12) gaführt sind.

6. Endoskop nach Anspruch 5,
**dadurch gekennzeichnet, daß** der weitere Übertragungskanal (8) durch einen in Richtung der Achse des Endoskop-Schaftes (2) verlaufenden Schlitz mit dem Hauptkanal (4) verbunden ist.

7. Endoskop nach Anspruch 5 oder 6,
**dadurch gekennzeichnet, daß** die Querschnitts-Außenkontur der Videoeinheit (1) derart der Innenkontur des Hauptkanals (4) angepaßt ist, daß diese durch den Hauptkanal (4) in die Beobachtungsstellung vorschiebbar und zum proximalen Ende zurückschiebbar ist.

8. Endoskop nach einem der Ansprüche 5 bis 7,
**dadurch gekennzeichnet, daß** der weitere Übertragungskanal (8) als Führung für das Bewegungselement (7) dient.

9. Endoskop nach Anspruch 7,
**dadurch gekennzeichnet, daß** an der Videoeinheit (1) exzentrisch bezogen auf den Querschnitt der Videoeinheit eine als Bewegungselement dienende Schubstange (7') angebracht ist.

10. Endoskop nach Anspruch 9,
**dadurch gekennzeichnet, daß** die Längsschnitts-Kontur der Videoeinheit (1) derart ausgebildet ist, daß sich ein kantenfreier und weicher Übergang vom maximalen Querschnitt der Videoeinheit zum Querschnitt der Schubstange (7') ergibt.

11. Endoskop nach Anspruch 10,
**dadurch gekennzeichnet, daß** die Schubstange (7') innerhalb des Hauptkanals (4) bewegbar ist.

12. Endoskop nach einem der Ansprüche 1 bis 11,
**dadurch gekennzeichnet, daß** das Bewegungselement (7) hohl ist, und daß im Bewegungselement das Übertragungssystem (12) geführt ist.

13. Endoskop nach Anspruch 12,
**dadurch gekennzeichnet, daß** das Bewegungselement (7; 7') ein starres Hohlrohr oder eine biegsame Welle ist.

14. Endoskop nach einem der Ansprüche 1 bis 13,
**dadurch gekennzeichnet, daß** die Videoeinheit (1) in der Ruhestellung in den Schaft (2) einschwenkbar ist.

15. Endoskop nach einem der Ansprüche 1 bis 14,
**dadurch gekennzeichnet, daß** wenigstens zwei bewegbare und insbesondere schwenkbare Einheiten (1',1") vorgesehen sind, von denen wenigstens eine eine Videoeinheit ist.

16. Endoskop nach Anspruch 15,
**dadurch gekennzeichnet, daß** die bewegbaren und insbesondere schwenkbaren Einheilen (1',1") hintereinander am Endoskopschaft (2) angeordnet sind.

17. Endoskop nach Anspruch 15 oder 16,
**dadurch gekennzeichnet, daß** die Einheiten (1', 1") nach dem Ausschwenken in einer auf der Endoskop-Längsachse senkrecht stehenden Ebene angeordnet sind.

18. Endoskop nach Anspruch 17,
**dadurch gekennzeichnet, daß** die beiden Einheiten (1', 1") Videoeinheiten sind, so daß eine Stereobetrachtung möglich ist.

19. Endoskop nach Anspruch 18,
**dadurch gekennzeichnet, daß** eine der beiden Einheiten eine Videoeinheit und die andere Einheit ein Lichtsender ist, so daß ein Triangulationsmeßverfahren möglich ist.

20. Endoskop nach Anspruch 19,
**dadurch gekennzeichnet, daß** eine der beiden Einheiten eine Videoeinheit ist und die andere Einheit ein anderen bildgebenden Aufnehmer aufweist.

21. Endoskop mit einer am distalen Ende eines EndoskopSchaftes angeordneten Videoeinrichtung, die mittels eines Übertragungssystems mit einer proximal angeordneten Versorgungseinheit verbunden ist, und die ein Objektiv aufweist, das ein mittels einer Beleuchtungseinheit beleuchtetes Objektfeld auf einen Bildaufnehmer abbildet, wobei das Objektiv (10) und der Bildaufnehmer (11) zu einer Videoeinheit (1) zusammengefaßt sind, wobei die Videoeinheit derart beweglich an dem Endoskop-Schaft gehalten ist, daß die Querschnitts-Außenkontur der Videoeinheit beim Einführen in den zu beobachtenden Hohlraum in wesentlichen innerhalb der Querschnitts-Außenkontur des distalen Endes des Endoskop-Schaftes liegt und nach Beendigung des Einführvorganges die Videoeinheit relativ zum distalen Ende des Endoskop-Schafts über die Querschnitts-Außenkontur hinaus bewegbar ist, **dadurch gekennzeichnet, daß** der Endoskop-Schaft einen Hauptkanal (4) aufweist und die Videoeinheit derart in den Hauptkanal eingesetzt ist, daß sie in diesem verschoben werden kann und, daß an der Videoeinheit (1) exzentrisch bezogen auf den Querschnitt der Videoeinheit eine als Bewegungselement dienende Schubstange (7') angebracht ist, wobei die Videoeinheit (1) in einer Richtung verschiebbar ist, die mit der Endoskop-Achse (2') einen Winkel ungleich 0° einschließt.

22. Endoskop nach einem der Ansprüche 1 bis 21,
**dadurch gekennzeichnet, daß** die Videoeinheit (1) vom distalen Ende des Endoskop-Schaftes (2) lösbar ist, wobei sie lediglich über das Übertragungssystem (12) mit der proximalen Versorgungseinheit verbunden bleibt, so daß sie als Videosonde verwendbar ist, und daß sie anschließend und insbesondere zur Entnahme des Endoskops aus dem Hohlraum wieder an dem distalen Ende anbringbar ist.

23. Endoskop nach Anspruch 22,
**dadurch gekennzeichnet, daß** die Videosonde (1) nach vorne aus dem Endoskopschaft (2) herausschiebbar ist.

24. Endoskop mit einer am distalen Ende eines EndoskopSchaftes angeordneten Videoeinrichtung, die mittels eines Übertragungssystems mit einer proximal angeordneten Versorgungseinheit verbunden ist, und die ein Objektiv aufweist, das ein mittels einer Beleuchtungseinheit beleuchtetes Objektfeld auf einen Bildaufnehmer abbildet, wobei das Objektiv (10) und der Bildaufnehmer (11) zu einer Videoeinheit (1) zusammengefaßt sind, wobei die Videoeinheit derart beweglich an dem Endoskop-Schaft gehalten ist, daß die Querschnitts-Außenkontur der Videoeinheit beim Einführen in den zu beobachtenden Hohlraum in wesentlichen innerhalb der Querschnitts-Außenkontur des distalen Endes des Endoskop-Schaftes liegt und nach Beendigung des Einführvorganges die Videoeinheit relativ zum distalen Ende des Endoskop-Schafts über die Querschnitts-Außenkontur hinaus bewegbar ist, **dadurch gekennzeichnet, daß** die Videoeinheit wenigstens zwei Bildaufnehmer (11', 11'') mit zugeordneten Objektiven (10', 10'') aufweist und um wenigstens eine zur Längsachse (2') des Endoskop-Schaftes (2) senkrechte Achse (20) drehbar bzw. schwenkbar ist.

25. Endoskop nach einem der Ansprüche 1 bis 24,
**dadurch gekennzeichnet, daß** in der Videoeinheit (1) der Bildaufnehmer (11) senkrecht zur Längsachse (2') des Endoskop-Schaftes (2) angeordnet ist (Fig. 1).

26. Endoskop nach einem der Ansprüche 1 bis 25,
**dadurch gekennzeichnet, daß** in der Videoeinheit (1) der Bildaufnehmer (11) parallel zur Achse (2') des Endoskop-Schaftes (2) angeordnet ist (Fig. 2).

27. Endoskop nach einem der Ansprüche 1 bis 26,
**dadurch gekennzeichnet, daß** einem Bildaufnehmer zwei Objektive zugeordnet sind, die deren Bild wahlweise auf den Bildaufnehmer gerichtet werden kann.

28. Endoskop nach einem der Ansprüche 1 bis 27,
**dadurch gekennzeichnet, daß** für jeden Farbauszug ein Bildwandler (11₁, 11₂, 11₃) vorgesehen ist, auf den ein dichroitisches Umlenksystern (14) das Licht lenkt.

29. Endoskop nach einem der Ansprüche 1 bis 28,
**dadurch gekennzeichnet, daß** das Übertragungssystem wenigstens ein Verbindungskabel (12) für elektrische und/oder optische Übertragung von Energie und Signalen aufweist.

30. Endoskop nach einem der Ansprüche 1 bis 29,
**dadurch gekennzeichnet, daß** die elektrischen Signal- und Energieleitungen und die Lichtleiter zu einem einzigen Verbindungskabel (12) zusammengefaßt sind.

31. Endoskop nach Anspruch 30 in Verbindung mit Anspruch 23,
**dadurch gekennzeichnet, daß** die Verschiebung mittels dem bzw. den Verbindungkabel(n) zwischen Videoeinheit und proximalen Endbereich erfolgt.

32. Endoskop nach einem der Ansprüche 1 bis 31,
**dadurch gekennzeichnet, daß** die Lichtaustrittsfläche der Beleuchtungseinheit (9) am Endoskop-Schaft (2) vorgesehen ist.

33. Endoskop nach einem der Ansprüche 1 bis 31,
**dadurch gekennzeichnet, daß** die Lichtaustrittsfläche der Beleuchtungseinheit (9) an der Videoeinheit (1) vorgesehen ist.

34. Endoskop nach Anspruch 32 oder 33,
**dadurch gekennzeichnet, daß** die Beleuchtungseinheit eine in der Videoeinheit angeordnete Beleuchtungslichtquelle aufweist.

35. Endoskop nach einem der Ansprüche 1 bis 33,
**dadurch gekennzeichnet, daß** die Beleuchtungseinheit eine proximal angeordnete Beleuchtungslichtquelle ausweist, deren Licht Lichtleiter (7,9) zur Lichtaustrittsfläche(n) leiten.

36. Endoskop nach einem der Ansprüche 1 bis 34,
**dadurch gekennzeichnet, daß** in der Videoeinheit optoelektrische und/oder elektromagnetische Wandler für die Energieversorgung des Bildaufnehmers sowie gegebenenfalls der Beleuchtungslichtquelle vorgesehen sind.

37. Endoskop nach einem der Ansprüche 1 bis 36,
**dadurch gekennzeichnet, daß** in der Videoeinheit ein optoelektrischer Signalwandler für das Ausgangssignal des Bildaufnehmers vorgesehen ist.

38. Endoskop nach einem der Ansprüche 23 bis 37,
**dadurch gekennzeichnet, daß** der dem proximale Seite zugekehrte Endbereich der Videoeinheit sich stetig verjüngt, und daß der distale Endbereich des Endoskopschaftes komplementär ausgebildet ist (Fig. 8).

39. Endoskop nach einem der Ansprüche 1 bis 38,
**dadurch gekennzeichnet, daß** der Endoskopschaft flexibel ausgebildet ist, und fluidbeaufschlagbare Kammern aufweist, die bei einer Beaufschlagung den Endoskopschaft gegen Querkräfte versteifen.

40. Endoskop nach einem der Ansprüche 1 bis 39,
**dadurch gekennzeichnet, daß** der Endoskop-Schaft entlang seiner Längsachse aufklappbar ist oder einen Längsschlitz aufweist, so daß die Videoeinheit vom Endoskopschaft trennbar ist.

41. Endoskop nach einem der Ansprüche 1 bis 40,
**dadurch gekennzeichnet, daß** die Videoeinheit ein magnetisch beeinflußbares Element zur berührungslosen Positionierung der Videoeinheit unabhängig vom Endoskopschaft aufweist.

42. Endoskop nach einem der Ansprüche 1 bis 41,
**dadurch gekennzeichnet, daß** das oder die Verbindungskabel Stecker aufweisen, deren Querschnittsabmessungen kleiner oder gleich den Abmessungen der Verbindungskabel sind, die wiederum wesentlich kleiner als die maximalen Querschnittsabmessungen der Videoeinheit sind.

43. Endoskop nach einem der Ansprüche 1 bis 42,
**dadurch gekennzeichnet, daß** die optische Achse des Objektivs der Videoeinheit mit der Achse des Endoskop-Schaftes einen Winkel einschließt.

44. Endoskop nach einem der Ansprüche 1 bis 43,
**dadurch gekennzeichnet, daß** die Videoeinheit wenigstens einen Spülkanal zum Spülen des Objektivs aufweist.

45. Endoskop nach einem der Ansprüche 1 bis 43,
**dadurch gekennzeichnet, daß** anstelle der Videoeinheit eine Beobachtungseinheit (1''') mit Bild-Weiterleitern einsetzbar ist.

46. Endoskop nach einem der Ansprüche 1 bis 45,
**dadurch gekennzeichnet, daß** die Beobachtungseinheit in den Hauptkanal einsetzbar ist.

47. Endoskop nach einem der Ansprüche 1 bis 46,
**dadurch gekennzeichnet, daß** der Querschnitt des Schaftes und der Video-Einheit oval ist, und daß die Videoeinheit um die Längsachse des Schaftes um 90° drehabr ist.

## Claims

1. Endoscope with a video device which is arranged at the distal end of an endoscope shaft, is connected by means of a transmission system to a proximally arranged supply unit, and has an objective which images an object field, illuminated by means of an illumination unit, on an image sensor, the objective (10) and the image sensor (11) being combined to form a video unit (1), the video unit being movably held on the endoscope shaft in such a way that the outer contour of the cross-section of the video unit lies substantially within the outer contour of the cross-section of the distal end of the endoscope shaft upon introduction into the cavity to be observed and is movable relative to the distal end of the endoscope shaft beyond the outer contour of the cross-section after completion of the introduction procedure, **characterised in that** the video unit is pivotable about an axis (3) parallel to the axis (2') of the endoscope shaft and eccentric in relation to the end face of the video unit (1).

2. Endoscope according to Claim 1, **characterised in that** the outside diameter of the cross-section of the video unit (1) is approximately as great as that of the endoscope shaft (2).

3. Endoscope according to Claim 1 or 2, **characterised in that** the video unit (1) is articulated on the distal end of the endoscope in a rotatably displaceable manner, and **in that** a control cable is provided for performing the pivoting movement, which cable runs from the proximal end to the distal end and back and the force of which is deflected from axial to lateral via a distal roller system.

4. Endoscope according to one of Claims 1 to 3, **characterised in that** the endoscope shaft (2) has, in a manner known per se, at least one main duct (4) for rinsing fluids, instruments etc., which connects the distal end to the proximal end, and **in that** the video unit (1) at least partially covers the duct opening during the procedure of introducing the shaft into the cavity and withdrawing it therefrom, and clears the main duct (4) in the observation position.

5. Endoscope according to one of Claims 1 to 4, **characterised in that** the endoscope shaft (2) has a transmission duct (8), in which the elements (movement elements 7) which produce the pivoting movement of the video unit, and, if necessary, the transmission system (12) are run.

6. Endoscope according to Claim 5, **characterised in that** the further transmission duct (8) is connected to the main duct (4) by a slot running in the direction of the axis of the endoscope shaft (2).

7. Endoscope according to Claim 5 or 6, **characterised in that** the outer contour of the cross-section of the video unit (1) is adapted to the inner contour of the main duct (4) in such a way that the said unit can be pushed forwards through the main duct (4) into the observation position and pushed back to the proximal end.

8. Endoscope according to one of Claims 5 to 7, **characterised in that** the further transmission duct (8) serves as a guide for the movement element (7).

9. Endoscope according to Claim 7, **characterised in that** a push rod (7') serving as a movement element is attached to the video unit (1) eccentrically in relation to the cross-section of the video unit.

10. Endoscope according to Claim 9, **characterised in that** the contour of the longitudinal section of the video unit (1) is designed in such a way that an edge-free and smooth transition from the maximum cross-section of the video unit to the cross-section of the push rod (7') results.

11. Endoscope according to Claim 10, **characterised in that** the push rod (7') is movable within the main duct (4).

12. Endoscope according to one of Claims 1 to 11, **characterised in that** the movement element (7) is hollow, and **in that** the transmission system (12) is run in the movement element.

13. Endoscope according to Claim 12, **characterised in that** the movement element (7; 7') is a rigid hollow tube or a flexible shaft.

14. Endoscope according to one of Claims 1 to 13, **characterised in that** the video unit (1) can be pivoted into the shaft (2) in the rest position.

15. Endoscope according to one of Claims 1 to 14, **characterised in that** at least two movable and in particular pivotable units (1', 1") are provided, at least one of which is a video unit.

16. Endoscope according to Claim 15, **characterised in that** the movable and in particular pivotable units (1', 1") are arranged one behind the other on the endoscope shaft (2).

17. Endoscope according to Claim 15 or 16, **characterised in that** after being pivoted out the units (1', 1") are arranged in a plane situated perpendicularly to the longitudinal axis of the endoscope.

18. Endoscope according to Claim 17, **characterised in that** the two units (1', 1") are video units, so that stereo observation is possible.

19. Endoscope according to Claim 18, **characterised in that** one of the two units is a video unit and the other unit is a light transmitter, so that a triangulation measurement method is possible.

20. Endoscope according to Claim 19, **characterised in that** one of the two units is a video unit and the other unit has another image-forming sensor.

21. Endoscope with a video device which is arranged at the distal end of an endoscope shaft, is connected by means of a transmission system to a proximally arranged supply unit, and has an objective which images an object field, illuminated by means of an illumination unit, on an image sensor, the objective (10) and the image sensor (11) being combined to form a video unit (1), the video unit being movably held on the endoscope shaft in such a way that the outer contour of the cross-section of the video unit lies substantially within the outer contour of the cross-section of the distal end of the endoscope shaft upon introduction into the cavity to be observed and is movable relative to the distal end of the endoscope shaft beyond the outer contour of the cross-section after completion of the introduction procedure, **characterised in that** the endoscope shaft has a main duct (4) and the video unit is inserted into the main duct in such a way that it can be displaced therein, and **in that** a push rod (7') serving as a movement element is attached to the video unit (1) eccentrically in relation to the cross-section of the video unit, the video unit (1) being displaceable in a direction which forms an angle not equal to 0° with the endoscope axis (2').

22. Endoscope according to one of Claims 1 to 21, **characterised in that** the video unit (1) is detachable from the distal end of the endoscope shaft (2), it remaining connected to the proximal supply unit merely via the transmission system (12), so that it is usable as a video probe, and **in that** it is subsequently reattachable to the distal end, in particular for withdrawal of the endoscope from the cavity.

23. Endoscope according to Claim 22, **characterised in that** the video probe (1) can be pushed forwards out of the endoscope shaft (2).

24. Endoscope with a video device which is arranged at the distal end of an endoscope shaft, is connected by means of a transmission system to a proximally arranged supply unit, and has an objective which images an object field, illuminated by means of an illumination unit, on an image sensor, the objective (10) and the image sensor (11) being combined to form a video unit (1), the video unit being movably held on the endoscope shaft in such a way that the outer contour of the cross-section of the video unit lies substantially within the outer contour of the cross-section of the distal end of the endoscope shaft upon introduction into the cavity to be observed and is movable relative to the distal end of the endoscope shaft beyond the outer contour of the cross-section after completion of the introduction procedure, **characterised in that** the video unit has at least two image sensors (11', 11") with assigned objectives (10', 10") and is rotatable or pivotable about at least an axis (20) perpendicular to the longitudinal axis (2') of the endoscope shaft (2).

25. Endoscope according to one of Claims 1 to 24, **characterised in that** the image sensor (11) is arranged in the video unit (1) perpendicularly to the longitudinal axis (2') of the endoscope shaft (2) (Fig. 1).

26. Endoscope according to one of Claims 1 to 25, **characterised in that** the image sensor (11) is arranged in the video unit (1) parallel to the axis (2') of the endoscope shaft (2) (Fig. 2).

27. Endoscope according to one of Claims 1 to 26, **characterised in that** an image sensor is assigned two objectives, the image from which can be selectively directed to the image sensor.

28. Endoscope according to one of Claims 1 to 27, **characterised in that** an image converter (11₁, 11₂, 11₃), to which the light is directed by a dichroic deflection system (14), is provided for each chromatic component.

29. Endoscope according to one of Claims 1 to 28, **characterised in that** the transmission system has at least one connection cable (12) for electrical and/or optical transmission of energy and signals.

30. Endoscope according to one of Claims 1 to 29, **characterised in that** the electrical signal and energy lines and the light guides are combined to form a single connection cable (12).

31. Endoscope according to Claim 30 in conjunction with Claim 23, **characterised in that** the displacement is effected between video unit and proximal end region by means of the connection cable(s).

32. Endoscope according to one of Claims 1 to 31, **characterised in that** the light exit area of the illumination unit (9) is provided on the endoscope shaft (2).

33. Endoscope according to one of Claims 1 to 31, **characterised in that** the light exit area of the illumination unit (9) is provided on the video unit (1).

34. Endoscope according to Claim 32 or 33, **characterised in that** the illumination unit has an illumination light source arranged in the video unit.

35. Endoscope according to one of Claims 1 to 33, **characterised in that** the illumination unit has a proximally arranged illumination light source, the light of which is guided to the light exit area(s) by light guides (7, 9).

36. Endoscope according to one of Claims 1 to 34, **characterised in that** optoelectrical and/or electromagnetic transducers for supplying energy to the image sensor and, if necessary, to the illumination light source are provided in the video unit.

37. Endoscope according to one of Claims 1 to 36, **characterised in that** an optoelectrical signal converter for the output signal of the image sensor is provided in the video unit.

38. Endoscope according to one of Claims 23 to 37, **characterised in that** the end region, facing the proximal side, of the video unit tapers continuously, and **in that** the distal end region of the endoscope shaft is of complementary design (Fig. 8).

39. Endoscope according to one of Claims 1 to 38, **characterised in that** the endoscope shaft is of flexible design, and has chambers which are capable of being supplied with fluid and, when supplied, stiffen the endoscope shaft against transverse forces.

40. Endoscope according to one of Claims 1 to 39, **characterised in that** the endoscope shaft can be swung open along its longitudinal axis or has a longitudinal slot, so that the video unit can be separated from the endoscope shaft.

41. Endoscope according to one of Claims 1 to 40, **characterised in that** the video unit has a magnetically influenceable element for contactless positioning of the video unit independently of the endoscope shaft.

42. Endoscope according to one of Claims 1 to 41, **characterised in that** the connection cable(s) has(have) plugs, the cross-sectional dimensions of which are less than or equal to the dimensions of the connection cables, which in turn are substantially less than the maximum cross-sectional dimensions of the video unit.

43. Endoscope according to one of Claims 1 to 42, **characterised in that** the optical axis of the objective of the video unit forms an angle with the axis of the endoscope shaft.

44. Endoscope according to one of Claims 1 to 43, **characterised in that** the video unit has at least one rinsing duct for rinsing the objective.

45. Endoscope according to one of Claims 1 to 43, **characterised in that** an observation unit (1''') with image forwarders can be used instead of the video unit.

46. Endoscope according to one of Claims 1 to 45, **characterised in that** the observation unit can be inserted into the main duct.

47. Endoscope according to one of Claims 1 to 46, **characterised in that** the cross-section of the shaft and of the video unit is oval, and **in that** the video unit is rotatable through 90° about the longitudinal axis of the shaft.

## Revendications

1. Endoscope à dispositif vidéo disposé à l'extrémité distale d'une tige (2) d'endoscope, le dispositif vidéo étant raccordé à une unité d'alimentation disposée du côté proximal, moyennant un système de transmission, et comprenant un objectif (10) qui produit, sur un enregistreur d'images (11), l'image d'un champ de l'objet éclairé moyennant une unité d'éclairage, l'objectif (10) et l'enregistreur d'images (11) étant combinés l'un avec l'autre de façon à former une unité vidéo (1), l'unité vidéo étant montée mobile sur la tige de l'endoscope de façon que le contour extérieur de la section transversale de l'unité vidéo se trouve essentiellement au-dedans du contour extérieur de l'extrémité distale de la tige de l'endoscope, quand elle est introduite dans la cavité à explorer, et qu'après la fin de l'opération de son introduction, l'unité vidéo soit mobile relativement à l'extrémité distale de la tige de l'endoscope en dehors dudit contour extérieur,
**caractérisé en ce que** l'unité vidéo est pivotable autour d'un axe (3) parallèle à l'axe (2') de la tige de l'endoscope et excentrique relativement à la face frontale de l'unité vidéo (1).

2. Endoscope selon la revendication 1,
**caractérisé en ce que** le diamètre extérieur de la section transversale de l'unité vidéo (1) est approximativement aussi grand que le diamètre extérieur de la tige (2) de l'endoscope.

3. Endoscope selon la revendication 1 ou 2,
**caractérisé en ce que** l'unité vidéo (1) est articulée à l'extrémité distale de l'endoscope pour être pivotable en rotation, et **en ce qu'**un système de commande par câble est prévu pour la réalisation du mouvement pivotant, lequel système s'étend de l'extrémité proximale à l'extrémité distale et retour, dont la force est renvoyée d'un sens axial à un sens latéral moyennant un système de galets distal.

4. Endoscope selon une quelconque des revendications 1 à 3,
**caractérisé en ce que** la tige (2) de l'endoscope comprend, de façon connue en soi, au moins un passage principal (4) pour des liquides de lavage, des instruments, etc. qui relie l'extrémité distale à l'extrémité proximale, et **en ce que** l'unité vidéo (1) couvre, au moins en partie, l'ouverture dudit passage au cours des opérations d'introduction et de sortie de la tige dans ou respectivement de la cavité, en dégageant, par contre, le passage principal (4) dans la position d'exploration.

5. Endoscope selon une quelconque des revendications 1 à 4,
**caractérisé en ce que** la tige (2) de l'endoscope comprend un passage de transmission (8) dans lequel s'étendent les éléments (éléments moteur 7) qui commandent le mouvement pivotant de l'unité vidéo, ainsi qu'éventuellement ledit système de transmission (12).

6. Endoscope selon la revendication 5,
**caractérisé en ce que** ledit autre passage de transmission (8) est raccordé audit passage principal (4) par une fente qui s'étend le long de l'axe de la tige (2) de l'endoscope.

7. Endoscope selon la revendication 5 ou 6,
**caractérisé en ce que** le contour extérieur de la section transversale de l'unité vidéo (1) est adapté au contour intérieur dudit passage principal (4) de façon que l'unité soit apte à être avancée par ledit passage principal (4) dans la position d'exploration et à être repoussée vers l'extrémité proximale.

8. Endoscope selon une quelconque des revendications 5 à 7,
**caractérisé en ce que** ledit autre passage de transmission (8) sert à guider l'élément moteur (7).

9. Endoscope selon la revendication 7,
**caractérisé en ce qu'**une bielle de commande (7') est montée sur l'unité vidéo (1) de façon excentrique, relativement à la section transversale de l'unité vidéo, laquelle bielle fait fonction d'élément moteur.

10. Endoscope selon la revendication 9,
**caractérisé en ce que** le contour de la section longitudinale de l'unité vidéo (1) est configuré de façon à former une transition sans arêtes et douce de la section transversale maximale de l'unité vidéo à la section transversale de la bielle de commande (7').

11. Endoscope selon la revendication 10,
**caractérisé en ce que** la bielle de commande (7') est mobile au dedans du passage principal (4).

12. Endoscope selon une quelconque des revendications 1 à 11,
**caractérisé en ce que** l'élément moteur (7) est creux, et **en ce que** le système de transmission (12) est guidé dans l'élément moteur.

13. Endoscope selon la revendication 12,
**caractérisé en ce que** l'élément moteur(7 ; 7') est un tube creux rigide ou un arbre flexible.

14. Endoscope selon une quelconque des revendications 1 à 13,
**caractérisé en ce que** l'unité vidéo (1) est disposée de façon à être pivotée dans la tige (2) en position de repos.

15. Endoscope selon une quelconque des revendications 1 à 14,
**caractérisé en ce qu'**au moins deux unités (1', 1'') mobiles et en particulier pivotables sont prévues, dont au moins une est une unité vidéo.

16. Endoscope selon la revendication 15,
**caractérisé en ce que** lesdites unités (1', 1'') mobiles et en particulier pivotables sont disposées, l'une derrière l'autre, sur la tige (2) de l'endoscope.

17. Endoscope selon la revendication 15 ou 16,
**caractérisé en ce qu'**après leur pivotement, lesdites unités (1', 1'') sont disposées dans un plan perpendiculaire sur l'axe longitudinal de l'endoscope.

18. Endoscope selon la revendication 17,
**caractérisé en ce que** lesdites deux unités (1', 1'') sont des unités vidéo, de façon qu'elles permettent une exploration stéréoscopique.

19. Endoscope selon la revendication 18,
**caractérisé en ce qu'**une desdites deux unités est une unité vidéo, et **en ce que** l'autre unité est un émetteur de lumière, de façon à permettre la réalisation d'un procédé de mesure à triangulation.

20. Endoscope selon la revendication 19,
**caractérisé en ce qu'**une desdites deux unités est une unité vidéo, et **en ce que** l'autre unité comprend un autre enregistreur à produire des images.

21. Endoscope à dispositif vidéo disposé à l'extrémité distale d'une tige (2) d'endoscope, le dispositif vidéo étant raccordé à une unité d'alimentation disposée du côté proximal, moyennant un système de transmission, et comprenant un objectif (10) qui produit, sur un enregistreur d'images (11), l'image d'un champ de l'objet éclairé moyennant une unité d'éclairage, l'objectif (10) et l'enregistreur d'images (11) étant combinés l'un avec l'autre pour former une unité vidéo (1), l'unité vidéo étant montée mobile sur la tige de l'endoscope, de façon que le contour extérieur de la section transversale de ladite unité vidéo se trouve sensiblement au-dedans du contour extérieur de l'extrémité distale de la tige de l'endoscope, quand elle est introduite dans la cavité à explorer, et qu'après la fin de l'opération de son introduction, l'unité vidéo soit mobile relativement à l'extrémité distale de la tige de l'endoscope en dehors dudit contour extérieur,
**caractérisé en ce que** la tige de l'endoscope présente un canal principal (4), et l'unité vidéo est incorporée dans le canal principal de façon à pouvoir coulisser dans celui-ci, et **en ce que** sur l'unité vidéo (1) est disposée, de façon excentrique par rapport à la section transversale de l'unité vidéo, une tige de poussée (7') servant d'élément de déplacement, moyennant quoi l'unité vidéo (1) peut être déplacée dans une direction qui inscrit un angle qui n'est pas égal à 0° avec l'axe de l'endoscope (2').

22. Endoscope selon une quelconque des revendications 1 à 21,
**caractérisé en ce que** l'unité vidéo (1) est prévue pour être détachée de l'extrémité distale de la tige (2) de l'endoscope, en restant reliée à ladite unité d'alimentation proximale seulement par ledit système de transmission (12), de façon qu'elle soit utilisable en tant que sonde vidéo, et **en ce qu'**elle peut être rattachée à l'extrémité distale ensuite, en particulier pour la sortie de l'endoscope de la cavité.

23. Endoscope selon la revendication 22,
**caractérisé en ce que** la sonde vidéo (1) est prévue pour être poussée vers l'avant hors de la tige (2) de l'endoscope.

24. Endoscope à dispositif vidéo disposé à l'extrémité distale d'une tige (2) d'endoscope, le dispositif vidéo étant raccordé à une unité d'alimentation disposée du côté proximal, moyennant un système de transmission, et comprenant un objectif (10) qui produit, sur un enregistreur d'images (11), l'image d'un champ de l'objet éclairé moyennant une unité d'éclairage, l'objectif (10) et l'enregistreur d'images (11) étant combinés l'un avec l'autre à former une unité vidéo (1), l'unité vidéo étant montée mobile sur la tige de l'endoscope, de façon que le contour extérieur de la section transversale de l'unité vidéo se trouve essentiellement au-dedans du contour extérieur de l'extrémité distale de la tige de l'endoscope, quand elle est introduite dans la cavité à explorer, et qu'après la fin de l'opération de son introduction, l'unité vidéo soit mobile relativement à l'extrémité distale de la tige de l'endoscope en dehors dudit contour extérieur,
**caractérisé en ce que** l'unité vidéo présente au moins deux enregistreurs d'images (11', 11'') avec des objectifs affectés (10', 10'') et peuvent être mis en rotation ou en pivotement autour d'au moins un axe (20) perpendiculaire à l'axe longitudinal (2') de la tige d'endoscope (2).

25. Endoscope selon une quelconque des revendications 1 à 24,
**caractérisé en ce que** ledit enregistreur d'images (11) dans l'unité vidéo (1) est disposé perpendiculairement sur l'axe longitudinal (2') de la tige (2) de l'endoscope.

26. Endoscope selon une quelconque des revendications 1 à 25,
**caractérisé en ce que** 1' enregistreur d'images (11) dans ladite unité vidéo (1) est disposé en parallèle à l'axe (2') de la tige (2) de l'endoscope.

27. Endoscope selon une quelconque des revendications 1 à 26,
**caractérisé en ce que** deux objectifs appartiennent à un enregistreur d'images, dont les images respectives peuvent être dirigées, à volonté, sur ledit enregistreur d'images.

28. Endoscope selon une quelconque des revendications 1 à 27,
**caractérisé en ce qu'**un transformateur d'image (11₁, 11₂, 11₃) respectif est prévu pour chaque extrait des couleurs, sur lequel un système déviateur dichroïque (14) dirige la lumière.

29. Endoscope selon une quelconque des revendications 1 à 28,
**caractérisé en ce que** ledit système de transmission comprend au moins un câble de connexion (12) pour la transmission électrique et/ou optique de l'énergie et des signaux.

30. Endoscope selon une quelconque des revendications 1 à 29, **caractérisé en ce que** lesdites lignes électriques de signaux et d'énergie sont combinées avec lesdits guides de lumière de façon à former un seul câble de connexion (12).

31. Endoscope selon la revendication 30 en combinaison avec la revendication 23,
**caractérisé en ce que** le coulissement est réalisé moyennant ledit ou respectivement lesdits câble(s) de connexion entre l'unité vidéo et la partie d'extrémité proximale.

32. Endoscope selon une quelconque des revendications 1 à 31,
**caractérisé en ce que** la surface de sortie de lumière de l'unité d'éclairage (9) est prévue sur la tige (2) de l'endoscope.

33. Endoscope selon une quelconque des revendications 1 à 31,
**caractérisé en ce que** la surface de sortie de lumière de l'unité d'éclairage (9) est prévue sur l'unité vidéo (1).

34. Endoscope selon la revendication 32 ou 33,
**caractérisé en ce que** l'unité d'éclairage comprend une source de lumière d'éclairage qui est disposée dans l'unité vidéo.

35. Endoscope selon une quelconque des revendications 1 à 33,
**caractérisé en ce que** l'unité d'éclairage comprend une source de lumière d'éclairage qui est disposée du côté proximal et dont la lumière est guidée par des guides de lumière (7, 9) vers la ou lesdites surface(s) de sortie de lumière.

36. Endoscope selon une quelconque des revendications 1 à 34,
**caractérisé en ce que** des transformateurs ou convertisseurs opto-électriques et/ou électromagnétiques sont prévus dans l'unité vidéo pour l'alimentation dudit enregistreur d'images, ainsi qu'éventuellement de la source de lumière d'éclairage, en énergie.

37. Endoscope selon une quelconque des revendications 1 à 36,
**caractérisé en ce qu'**un convertisseur de signaux électro-optique est prévu dans l'unité vidéo pour la conversion du signal de sortie dudit enregistreur d'images.

38. Endoscope selon une quelconque des revendications 23 à 37,
**caractérisé en ce que** la zone terminale de l'unité vidéo, qui est tournée face à l'extrémité proximale, se réduit de façon continue, et **en ce que** la zone terminale distale de la tige de l'endoscope présente une forme complémentaire (fig. 8).

39. Endoscope selon une quelconque des revendications 1 à 38,
**caractérisé en ce que** la tige de l'endoscope a une structure flexible et comprend des chambres aptes à être soumises à l'action d'un fluide, lesquelles, en réponse à leur commande, raidissent la tige de l'endoscope contre des forces transversales.

40. Endoscope selon une quelconque des revendications 1 à 39,
**caractérisé en ce que** la tige de l'endoscope est ouvrable le long de son axe longitudinal, ou comprend une fente longitudinale, de façon à isoler l'unité vidéo de ladite tige de l'endoscope.

41. Endoscope selon une quelconque des revendications 1 à 40,
**caractérisé en ce que** l'unité vidéo comprend un élément apte à être soumis à une influence magnétique pour un positionnement sans contact de ladite unité vidéo, indépendamment de la tige de l'endoscope.

42. Endoscope selon une quelconque des revendications 1 à 41,
**caractérisé en ce que** ledit ou lesdits câble(s) de connexion comprend (comprennent) des connecteurs dont les dimensions de la section transversale sont égales aux dimensions desdits câbles de connexion, ou plus petites que ces dernières, les dimensions desdits câbles étant encore essentiellement plus petites que ces dimensions maximales de la section transversale de l'unité vidéo.

43. Endoscope selon une quelconque des revendications 1 à 42,
**caractérisé en ce que** l'axe optique de l'objectif de ladite unité vidéo forme un angle avec l'axe de la tige de l'endoscope.

44. Endoscope selon une quelconque des revendications 1 à 43,
**caractérisé en ce que** l'unité vidéo comprend au moins un passage de lavage pour nettoyer l'objectif.

45. Endoscope selon une quelconque des revendications 1 à 43,
**caractérisé en ce qu'**au lieu de l'unité vidéo, une unité d'observation (1''') avec des guides de transmission d'images est utilisable.

46. Endoscope selon une quelconque des revendications 1 à 45,
**caractérisé en ce que** l'unité d'observation est prévue à être introduite dans le passage principal.

47. Endoscope selon une quelconque des revendications 1 à 46,
**caractérisé en ce que** la section transversale de la tige et de l'unité vidéo est ovale, et **en ce que** ladite unité vidéo est rotative par 90° autour de l'axe longitudinal de ladite tige.
